# EUROPEAN PATENT SPECIFICATION

(11) **EP 3 231 832 B1**
(45) Date of publication and mention of the grant of the patent: **31.03.2021**
(21) Application number: 15881089.5
(22) Date of filing: 04.02.2015
(51) Int. Cl.: C08J 3/12, C08J 3/03

(54) **METHOD FOR PRODUCING MOLECULAR ASSEMBLIES**
VERFAHREN ZUR HERSTELLUNG VON MOLEKULAREN ANORDNUNGEN
PROCÉDÉ DE PRODUCTION D'ENSEMBLES MOLÉCULAIRES

(43) Date of publication of application: 18.10.2017
(73) Proprietor: Shimadzu Corporation, Kyoto-shi, Kyoto 604-8511 (JP)
(72) Inventor: MATSUTANI, Eri, Kyoto-shi Kyoto 604-8511 (JP); OZEKI, Eiichi, Kyoto-shi Kyoto 604-8511 (JP); KAWABE, Takashi, Kyoto-shi Kyoto 604-8511 (JP)
(74) Representative: Mewburn Ellis LLP
(86) International application number: PCT/JP2015/053116
(87) International publication number: WO 2016/125272

(56) References cited:
- EP-A1- 2 305 214
- EP-A1- 2 725 053
- EP-A1- 2 745 850
- WO-A1-2009/148121
- WO-A1-2012/176885
- JP-A- 2005 520 872
- JP-A- 2009 510 109
- JP-A- 2011 062 586
- JP-A- 2014 156 555
- DATABASE WPI Week 201442 Thomson Scientific, London, GB; AN 2014-K88559 XP002782905, & JP 2014 105161 A (SHIMADZU CORP) 9 June 2014 (2014-06-09)

## Description

### Technical Field

The present invention relates to a method for producing molecular assemblies of an amphiphilic block polymer having a hydrophilic block chain and a hydrophobic block chain.

### Technical Background

In recent years, there has been a growing interest in nanotechnology. New functional materials that take advantage of properties peculiar to nano-sized substances have been developed. Patent Document 1 discloses an amphiphilic block polymer having a hydrophilic block chain that includes a sarcosine unit and a hydrophobic block chain that includes a lactic acid unit. Patent Document 2 discloses a branched amphiphilic block polymer having a hydrophilic block chain of a branched structure that includes a sarcosine unit and a hydrophobic block chain that includes a lactic acid unit.

These amphiphilic block polymers self-organize in water and form molecular assemblies (lactosomes) such as micelles and vesicles having a particle size of about 10 nm to 500 nm. By using a hydrophobic polymer in addition to an amphiphilic block polymer, a volume of a hydrophobic core of molecular assemblies can be controlled and a size (particle size) of the molecular assemblies can also be adjusted.

Lactosomes exhibit high retentivity in blood, and an amount of lactosomes accumulated in liver is significantly low as compared to other molecular assemblies. Further, lactosomes can encapsulate a signal agent (such as a fluorescent agent), a ligand, a drug and the like, and can hold these substances on a surface by intermolecular interactions. Therefore, by utilizing a property (EPR effect) that nanoparticles having a particle size in a range from several tens of nanometers to several hundreds of nanometers retained in blood are likely to be accumulated in cancer, lactosomes can be used as nano-carriers for molecular imaging or drug delivery targeting a cancer site.

In Patent Document 1 and Patent Document 2, as methods for forming lactosomes from a solution of an amphiphilic block polymer, a "film method" and an "injection method" are described. In the film method, first, a solution containing an amphiphilic block polymer is prepared in a container such as a test tube or a flask. Next, a solvent is removed from the solution and a film of the amphiphilic block polymer is formed on an inner wall of the container. By adding water or an aqueous solution (water-based liquid) to the container and performing heating or an ultrasonic treatment, molecular assemblies in a water-base liquid are obtained. In the injection method, after an amphiphilic block polymer solution is dispersed in a water-based liquid, an organic solvent is removed.

Patent Document 3 describes a nanoparticle comprising an amphiphilic block polymer comprising a hydrophilic block having a sarcosine unit and a hydrophobic block having a lactic acid unit, and a substance labeled with a β-ray emitting nuclide for internal radiation therapy.

Patent Document 4 describes a nanoparticle comprising an amphiphilic block polymer which has a hydrophilic block having a sarcosine unit and a hydrophobic block having a lactic acid unit; and a metal ion coordinated in the polymer.

### Related Art

### Patent Document

Patent Document 1: International Publication No. WO 2009/148121.
   (family member of EP 2 305 214 A). .
Patent Document 2: International Publication No. WO 2012/176885.
Patent Document 3 European Publication No. EP 2745850
Patent Document 4 Japanese Publication No. JP 2014-105161

### Summary of the Invention

### Problems to Be Solved by the Invention

The above-described film method is a batch type production method and mass productivity is likely to be insufficient. Further, it is also difficult to make particle sizes uniform between different batches. In the film method, even when a volume of the container is increased in order to increase a production volume per batch, an increase in a surface area of the inner wall on which the film is formed is limited, and improvement in mass productivity is limited. Further, when an amount of a solution per one batch is increased or solution concentration is increased, when an organic solvent in the container is removed, a concentrated solution accumulates at a bottom of the container and a polymer is likely to precipitate in the solution. Therefore, problems may occur such as that formation of molecular assemblies is inhibited and that particle sizes become nonuniform.

The above-described injection method is also a batch type method and thus there are problems related to improvement in mass productivity and uniformity of particle sizes between batches. Further, after an amphiphilic block polymer solution is dispersed in a water-based liquid and molecular assemblies are obtained, it is necessary to remove an organic solvent contained in the solution. However, when a processing load per batch is increased, removal efficiency of the organic solvent decreases. Therefore, there is a limit to the improvement in mass productivity using a batch type production method.

In view of the above-describe situation, the present invention is intended to provide a molecular assembly production method for producing molecular assemblies containing an amphiphilic block polymer such as lactosomes with uniform particle size at high productivity, as defined in the claims.

### Means for Solving the Problems

In the present invention, a polymer solution containing a block polymer, which has a hydrophilic block chain and a hydrophobic block chain, and a solvent is applied in a layered shape on a planar base member and is dried, and thereby a polymer film is formed on the base member; and, by immersing the base member in a water-based liquid, molecular assemblies are obtained. In the amphiphilic block polymer, the hydrophilic block chain has 20 or more sarcosine units, and the hydrophobic block chain has 10 or more lactic acid units. As the solvent of the polymer solution, an organic solvent having a boiling point of 200 °C or less is preferably used. According to the method of the present invention, molecular assemblies having a particle size of, for example, about 10 to 500 nm are obtained.

As the base member, a flexible film used. When a flexible long film is used as a base member, since the processes including film formation, drying and molecular assembly formation can be continuously performed while the base member is moved, productivity of the molecular assemblies is increased.

When the polymer solution is applied in a layered shape, the base member preferably has a planar shape or a coating surface of the base member preferably has a convex curved surface shape. When the polymer solution is applied in a layered shape, the base member may be heated before the polymer solution is applied.

In addition to the amphiphilic block polymer, the polymer solution may contain a hydrophobic polymer, a signal agent, a ligand, a drug, or the like. Further, the polymer solution may contain a hydrophobic polymer to which a signal agent, a ligand, a drug, or like is bound. These addition compounds are incorporated into the molecular assemblies, and can be responsible for particle size control of the molecular assemblies and for function development. Further, by blending a signal agent, a ligand, a drug, or the like in the water-based liquid, these substances can also be incorporated into the molecular assemblies.

Also disclosed herein is a molecular assembly production apparatus usable in the above-described molecular assembly production method. The apparatus includes: a film forming part in which a polymer solution is applied in a layered shape on a planar base member, the polymer solution containing an amphiphilic block polymer and a solvent, the amphiphilic block polymer having a hydrophilic block chain and a hydrophobic block chain; a drying part in which a polymer film is formed on the base member by removing the solvent from the coated layer of the polymer solution; and a molecular assembly forming part in which molecular assemblies are obtained by bringing the polymer film into contact with a water-based liquid. The apparatus further includes a base member moving mechanism that sequentially moves the base member to the film forming part, the drying part and the molecular assembly forming part.

### Effect of Invention

In the method of the present invention, the amphiphilic block polymer solution is applied in a layered shape on the planar base member, and thus, solution accumulation is suppressed. Therefore, problems such as polymer precipitation in a concentrated solution are suppressed, and molecular assemblies excellent in particle size uniformity are obtained. Further, a series of processes from the application of the polymer solution to the formation of the molecular assemblies in the water-based liquid can be continuously performed. Therefore, productivity of the molecular assemblies is increased.

### Brief Description of Drawings

Fig. 1 is a schematic cross-sectional view illustrating a molecular assembly production apparatus for use in producing molecular assemblies according to the method of the invention.
Fig. 2 is a schematic cross-sectional view illustrating a molecular assembly production apparatus.

### Mode for Carrying Out the Invention

The present invention relates to a method for producing molecular assemblies of an amphiphilic block polymer. The amphiphilic block polymer is a block polymer having a hydrophilic block chain and a hydrophobic block chain, and self-organizes when in contact with a water-based liquid (water or an aqueous solution) and forms nanoparticles of molecular assemblies. The nanoparticles have a particle size of, for example, about 10 nm to 500 nm, and the particle size is adjusted according to an intended use. Examples of shapes of the molecular assemblies include micelle, vesicle and the like.

When the amphiphilic block polymer is in contact with a water-based liquid and the hydrophobic block chain forms a core, the hydrophilic block chain faces outward and molecules self-organize and form micelles. During the formation of the micelles, by allowing a drug or the like to coexist, the substance can be encapsulated inside the micelles, and a surface layer of the micelles and the substance can interact with each other. Further, during the formation of the micelles, by allowing a hydrophobic polymer or the like to coexist, a volume and properties of a hydrophobic core part can be changed, and the particle size of the micelles and a content rate of the drug, and the like, can be controlled.

The amphiphilic block polymer gathers to form a membrane shape with the hydrophobic block chain facing inward, and there are also cases where vesicles are formed having a structure in which the membrane-like body is closed to form a spherical shell shape. Usually, an internal hollow space of the vesicles is filled with an aqueous phase, and a drug or the like can be encapsulated in this aqueous phase. Further, a hydrophilic part of a membrane surface of the vesicles and a drug or the like can be caused to interact with each other.

In the present invention, a polymer solution containing an amphiphilic block polymer and a solvent is applied in a layered shape on a planar base member and is dried, and a polymer film is formed on the base member. By bringing the polymer film into contact with a water-based liquid, molecular assemblies of the amphiphilic polymer are obtained.

### [Amphiphilic Block Polymer]

An amphiphilic block polymer used in the present invention is a block polymer having a hydrophilic block chain and a hydrophobic block chain. Examples of monomer units of the hydrophilic block chain include alkylene oxide, sarcosine, and the like. Examples of monomer units of the hydrophobic block chain include hydroxy acids such as glycolic acid, lactic acid and hydroxyisobutyric acid, and hydrophobic amino acids or amino acid derivatives such as glycine, alanine, valine, leucine, isoleucine, proline, methionine, tyrosine, tryptophan, methyl glutamate, benzyl glutamate, methyl aspartate, ethyl aspartate and benzyl aspartate.

Among the exemplified amphiphilic block polymers, those in which the hydrophilic block chain has a sarcosine unit and the hydrophobic block chain has a lactic acid unit are used. In particular, an amphiphilic polymer in which the hydrophilic block chain has 20 or more sarcosine units and the hydrophobic block chain has 10 or more lactic acid units tends to form nanoparticles with uniform particle size, which can be suitably used as nano-carriers for molecular imaging or drug delivery targeting a cancer site or the like.

In the following, as an example of the amphiphilic block polymer, an amphiphilic block polymer that has a hydrophilic block chain having a sarcosine unit and a hydrophobic block chain having a lactic acid unit is described. The amphiphilic block polymer may be linear or branched. The hydrophilic block and the hydrophobic block are bound to each other via a linker.

### (Hydrophilic Block Chain)

The hydrophilic block chain contains a sarcosine unit (N-methylglycine unit). Sarcosine is highly soluble in water. Further, since polysarcosine has N-substituted amide, cis-trans isomerization is possible; and since there is less steric hindrance around an α carbon atom, polysarcosine is high flexible. Therefore, by using a polysarcosine chain as a structural unit, a hydrophilic block chain having both high hydrophilicity and flexibility is formed.

The hydrophilic block chain contains 20 or more sarcosine units. When the number of the sarcosine units is 20 or more, adjacent hydrophilic blocks of the block polymer are likely to aggregate, and self-cohesiveness is enhanced, and thus molecular assemblies such as micelles and vesicles are easily formed. An upper limit for the number of the sarcosine units in the hydrophilic block chain is not particularly limited. However, from a point of view of stabilizing structures of the molecular assemblies, the number of the sarcosine units is preferably 300 or less. The number of the sarcosine units in the hydrophilic block is more preferably 30 to 200, and even more preferably from 50 to 100.

In the hydrophilic block chain, all the sarcosine units may be continuous, or the sarcosine units may also be discontinuous as long as the above-described properties of the polysarcosine are not impaired. When the hydrophilic block chain has a monomer unit other than sarcosine, the monomer unit other than sarcosine is not particularly limited. Examples of monomer units other than sarcosine include hydrophilic amino acids or amino acid derivatives. The amino acids include α-amino acids, β-amino acids, and γ-amino acids, among which α-amino acids are preferable. Examples of hydrophilic α-amino acids include serine, threonine, lysine, aspartic acid, glutamic acid and the like. Further, the hydrophilic block may have a sugar chain, polyether, or the like. The hydrophilic block preferably has a hydrophilic group such as a hydroxyl group at a terminal (a terminal on an opposite side of the linker part with the hydrophobic block).

The hydrophilic block chain may have a linear or branched structure. When the hydrophilic block chain has a branched structure, each branch chain preferably contains 2 or more sarcosine units.

### (Hydrophobic Block Chain)

The hydrophobic block contains a lactic acid unit. Polylactic acid has excellent biocompatibility and stability. Further, polylactic acid has excellent biodegradability, and thus can be quickly metabolized and has low accumulation in other tissues than cancer tissue in vivo. Therefore, molecular assemblies obtained from an amphiphilic polymer using polylactic acid as a building block are useful for applications to living bodies, especially human bodies. Further, since polylactic acid has high solubility in low boiling point solvents, a low boiling organic solvent can be used for a solution (amphiphilic block polymer solution) for producing molecular assemblies. Therefore, production efficiency of the molecular assemblies is improved.

The hydrophobic block chain contains 10 or more lactic acid units. When the number of the lactic acid units is 10 or more, a hydrophobic core is easily formed, and self-cohesiveness is enhanced, and thus, molecular assemblies such as micelles and vesicles are easily formed. An upper limit for the number of the lactic acid units in the hydrophobic block chain is not particularly limited. However, from a point of view of stabilizing structures of the molecular assemblies, the number of the lactic acid units is preferably 300 or less. The number of the lactic acid units in the hydrophobic block is more preferably 20 to 200, and even more preferably from 30 to 100.

The lactic acid unit forming the hydrophobic block chain may be an L-lactic acid or a D-lactic acid. Further, the L-lactic acid and the D-lactic acid may be mixed. In the hydrophobic block chain, all the lactic acid units may be consecutive, or the lactic acid unit may also be discontinuous. Monomer units other than lactic acids contained in the hydrophobic block chain are not particularly limited. Examples of monomer units other than lactic acids include hydroxy acids such as glycolic acid and hydroxyisobutyric acid, and hydrophobic amino acids or amino acid derivatives such as glycine, alanine, valine, leucine, isoleucine, proline, methionine, tyrosine, tryptophan, glutamic acid methyl ester, glutamic acid benzyl ester, aspartic acid methyl ester, aspartic acid ethyl ester, and aspartic acid benzyl ester.

The hydrophobic block chain may have a linear or branched structure. When the hydrophobic block chain is not branched, during the formation of the molecular assemblies, a compact hydrophobic core is easily formed, and density of the hydrophilic block chain tends to increase. Therefore, in order to form core/shell type molecular assemblies having a small particle size and high structural stability, the hydrophobic block chain is preferably linear.

### (Structure and Synthesis Method of Amphiphilic Block Polymer)

An amphiphilic polymer is formed by causing a hydrophilic block chain and a hydrophobic block chain to bind to each other. The hydrophilic block chain and the hydrophobic block chain may be bound to each other via a linker. As the linker, it is preferable to use a substance having a functional group (such as a hydroxyl group or an amino group) capable of binding to a lactic acid monomer (lactic acid or lactide) (which is a structural unit of the hydrophobic block chain) or a polylactic acid chain, and a functional group (such as an amino group) capable of binding to a sarcosine monomer (such as sarcosine or N-carboxysarcosine anhydride) (which is a structural unit of the hydrophilic block) or polysarcosine. By appropriately selecting a linker, a branched structure of the hydrophilic block chain or the hydrophobic block chain can be controlled.

In the amphiphilic block polymer, the number of the sarcosine units contained in the hydrophilic block chain and the number of lactic acid units contained in the hydrophobic block chain are adjusted such that the amphiphilic block polymer can self-organize in a water-based liquid and form molecular assemblies. In the amphiphilic block polymer, a ratio (NS/NL) of the number (NS) of the sarcosine units to the number (NL) of the lactic acid units is preferably 0.05 to 10. The ratio (NS/NL) is preferably 0.5 to 7.5, and more preferably 1 to 5.

A synthesis method of an amphiphilic block polymer is not particularly limited. The commonly known peptide synthesis method, polyester synthesis method, depsipeptide synthesis method, and the like can be used. Specifically, an amphiphilic block polymer can be synthesized with reference to WO 2009/148121 (Patent Document 1) or WO 2012/176885 (Patent Document 2).

In order to more easily control a shape and a size of molecular assemblies, it is preferable to adjust a chain length of a polylactic acid in the hydrophobic block chain. In order to facilitate control of a chain length of a polylactic acid, when an amphiphilic block polymer is synthesized, it is preferable to first synthesize a polylactic acid having a linker introduced at one terminal and thereafter introduce a polysarcosine. By adjusting conditions such as a charging ratio of an initiator and a monomer in a polymerization reaction, a reaction time, temperature, and the like, chain lengths of a polysarcosine chain and a polylactic acid chain can be adjusted. The chain lengths of the hydrophilic block chain and the hydrophobic block chain (molecular weight of the amphiphilic block polymer) can be confirmed, for example, by using ¹H-NMR.

### [Production of Molecular Assemblies]

In the present invention, a solution containing an amphiphilic block polymer in an organic solvent is applied in a layered shape on a planar base member, and the coated layer on the base member is dried to form a film. By bringing the film into contact with a water-based liquid, molecular assemblies are obtained.

### (Organic Solvent)

The amphiphilic block polymer solution can be prepared by dissolving the amphiphilic block polymer in an organic solvent. The organic solvent is not particularly limited as long as it can dissolve amphiphilic block polymer. Examples of the organic solvent include: alcohols such as methanol, ethanol, isopropanol, butanol, trifluoroethanol, and hexafluoroisopropanol; ketones such as acetone, methyl ethyl ketone, methyl isobutyl ketone, methyl n-amyl ketone, cyclohexanone, diacetone alcohol, diisobutyl ketone, and methylcyclohexanone; chain ethers such as dimethyl ether, diethyl ether, methyl ethyl ether, methyl cellosolve, and methyl carbitol; cyclic ethers such as tetrahydrofuran, 1,2-dioxolane, 1,3-dioxolane, 1,2-dioxane, 1,3-dioxane, 1,4-dioxane, and 1,3,5-trioxane; aromatic hydrocarbons such as toluene and xylene; halogenated hydrocarbons such as chloroform, dichloromethane, dichloroethane, trichloroethane, and tetrachloroethane; esters such as methyl acetate, ethyl acetate, propyl acetate, isopropyl acetate, butyl acetate, ethyl lactate, butyl lactate, ethyl benzoate, and methyl acetoacetate; alicyclic hydrocarbons such as cyclopentane, cyclohexane, methylcyclohexane and ethylcyclohexane; and acetonitrile, dimethylsulfoxide, dimethylformamide, and the like. For example, in the formation of molecular assemblies (lactosomes) using an amphiphilic block polymer that has a hydrophilic block chain containing a sarcosine unit and a hydrophobic block chain containing a lactic acid unit, organic solvents such as alcohols, halogenated hydrocarbons, and dimethylformamides are preferably used. The organic solvent may be a mixed solvent composed of two or more solvents.

A type of an organic solvent used to prepare an amphiphilic block polymer solution can be selected according to a structure, a molecular weight, and the like of the polymer. In order to form micelles having a uniform particle size, a solvent having a high solubility for the polymer is preferably used. From a point of view of increasing a drying speed of a solvent and improving productivity, an organic solvent having a boiling point of 200 °C or less is preferably used. The boiling point of the organic solvent is more preferably 100 °C or less, and even more preferably 90 °C or less. When the molecular assemblies are applied to human bodies as nano-carriers for molecular imaging or drug delivery, an organic solvent such as an alcohol having less influence on human bodies is preferably used.

### (Addition Components in Block Polymer Solution)

The amphiphilic block polymer solution may contain substances other the amphiphilic block polymer and the solvent. For example, by including hydrophobic polymer in the solution, it is possible to promote formation of a hydrophobic core during formation of molecular assemblies and to adjust a particle size of the molecular assemblies. Further, by including a drug or the like in the solution, the dug or the like can be incorporated into the molecular assemblies.

The hydrophobic polymer has functions such as promoting the formation of the hydrophobic core and adjusting the size (particle size) of the molecular assemblies. That is, by allowing an amphiphilic block polymer and a hydrophobic polymer to coexist, a volume of a hydrophobic core in molecular assemblies can be increased and a particle size of the molecular assemblies can be controlled. By adjusting a molecular weight and a content of the hydrophobic polymer blended in the amphiphilic block polymer solution, the size of molecular assemblies can be adjusted. The number of structural units of the hydrophobic polymer is not particularly limited. However, in order to promote the formation of the hydrophobic core and to control the size of the molecular assemblies, a hydrophobic polymer having 10 or more lactic acid units is used. The number of lactic acid units of the hydrophobic polymer is more preferably 15 or more. From a point of view of achieving both size control by the hydrophobic polymer and structural stability of the molecular assemblies, the number of lactic acid units of the hydrophobic polymer is preferably 20 to 300, more preferably 25 to 200, and even more preferably 30 to 100.

The hydrophobic polymer may have other structural units than the lactic acid unit. As the structural units other than the lactic acid, those exemplified above as the structural units of the hydrophobic block such as hydroxy acid, hydrophobic amino acid or amino acid derivatives are preferably used.

In block polymer solution, a signal agent, a ligand, a drug or the like may be included. Further, a signal group, a ligand, a drug or the like can be bound to the hydrophobic polymer and used. A signal agent is a compound that contains a signal group, and imaging is enabled by detection of the signal group. Examples of signal groups include a fluorescent group, a radioactive element-containing group, a magnetic group, and the like. Examples of ligands include a ligand intended for targeting in order to allow molecular assemblies to be specifically bound to a target site when the molecular assemblies are administered to a living body, and a ligand for coordinating a signal agent or the like. Examples of the ligand intended for targeting include antibodies, adhesion factors such as arginine-glycine-aspartic acid (RGD), and the like. Examples of the ligand for coordinating a drug, a signal agent and the like to be carried to a target site include tricarboxylic acid capable of coordinating a transition metal and the like. Examples of the drug include drugs to be carried to a target site (target disease or the like) such as an anticancer agent, an antibacterial agent, an antiviral agent, an anti-inflammatory agent, an immunosuppressive agent, a steroid drug, a hormonal agent, and an angiogenesis inhibitor. Specific examples of anticancer agents include camptothecin, exatecan (camptothecin derivative), gemcitabine, doxorubicin, irinotecan, SN-38 (irinotecan active metabolite), 5-FU, cisplatin, oxaliplatin, paclitaxel, docetaxel and the like. It is possible that two or more of these drugs are used in combination.

When signal agents, ligands, drugs, and the like are bound to the hydrophobic polymer, the number of signal agents, ligands, drugs and the like bound to one polymer may be 1, or 2 or more. Here, "binding" between the hydrophobic polymer and a signal group, a ligand, a drug or the like specifically refers to a covalent bond, and includes both a form in which the signal group, the ligand, the drug or the like is directly bonded to a specific side of the hydrophobic polymer, and a form in which the signal group, the ligand, the drug or the like is indirectly bonded to a specific side of the hydrophobic polymer via a spacer group or the like. A spacer group used in the binding between the hydrophobic polymer and a signal agent, a ligand, a drug or the like is not particularly limited. Examples of spacers include: alkyl groups; polysaccharides such as carboxyl methyl cellulose and amylose; water soluble polymers such as polyalkylene oxide chains, polyethylene glycol chains, and polyvinyl alcohol chains; and the like.

A binding site of a signal agent, a ligand, a drug or the like can be any part of the hydrophobic polymer. When the hydrophobic polymer is a polylactic acid, a signal agent, a ligand, a drug or the like may be bound to a terminal structural unit of the polylactic acid or to an internal structural unitof the polylactic acid. In either case, the signal agent, the ligand, the drug or the like is held inside the molecular assemblies. When the molecular assemblies are micelles, micelles can be formed that hold a signal agent, a ligand, a drug or the like near a hydrophobic core inside the micelles and a boundary between a hydrophilic part and a hydrophobic part. When the molecular assemblies are vesicles, vesicles can be formed that encapsulate a signal agent, a ligand, a drug or the like in a membrane.

In addition to binding to a hydrophobic polymer, a signal agent, a ligand, a drug or the like can also be bound to other polymers and the like and be contained in the molecular assemblies. For example, it is also possible that a signal agent, a ligand, a drug or the like is bound to the hydrophobic block chain, the hydrophilic block chain, the linker or the like of the amphiphilic block polymer. In addition to being covalently bound to a polymer, a signal agent, a ligand, a drug or the like can also be encapsulated in the molecular assemblies or be incorporated into the molecular assemblies by intermolecular interactions with a surface layer part of the molecular assemblies.

Concentration of a solid component (the amphiphilic block polymer, the hydrophobic polymer, the signal agent, the ligand, the drug or the like) of the block polymer solution is not particularly limited. From a point of view of increasing drying efficiency after the solution is applied, the concentration of the solid component of the solution is preferably high. On the other hand, when the concentration of the solution is excessively high, problems such as polymer precipitation may occur. Taking these factors into consideration, the solid component concentration may be set according to a type or the like of the organic solvent. The solid component concentration of the block polymer solution is, for example, about 0.1 to 20 weight%.

### (Base Member)

The amphiphilic block polymer solution is applied in a layered shape on a base member and is dried, and thereby, a block polymer film is obtained. In order to apply the solution in a layered shape, a coating surface of the base member is planar. The term "planar" means that a portion where the solution is applied is planer. Base members disclosed herein may have a plate-like or cylindrical shape. Examples of a plate-shaped base member include rigid base members such as a glass plate, a resin plate and a metal plate, and flexible base members such as a resin film and a metal foil. The term "cylindrical" means endless and is not limited to a cylindrical shape. Examples of a cylindrical base member include a flexible base member such as an endless belt and a rigid base member such as a cylindrical cast drum roll. In order to increase continuous productivity, a flexible plate-shaped base member or a flexible or rigid cylindrical base member is preferably used.

A rigid base member may have a plate-like (planar) shape or a curved surface shape that is curved such that a coating surface of the base member is convex. When a base member is flexible, when the polymer solution is applied, the base member preferably has a planar shape or a curved surface shape that is curved such that a coating surface of the base member is convex.

In a method such as the conventional film method in which a solvent in a container is dried and a film is formed on a container inner wall surface, since the inner wall surface is concave, a solution concentrated during the removal of the solvent accumulates at a bottom of the container and polymer precipitation is likely to occur. In contrast, when a planar base member is used and a coating surface of the base member is planar or a coating surface side of the base member is in a convex curved surface shape, solution accumulation does not occur and the solution can be applied in a uniform layered shape on the base member. Therefore, polymer precipitation during drying is unlikely to occur, and molecular assemblies having a uniform particle size can be easily obtained. Further, as compared to the case of forming a film on a container inner wall surface, since a contact surface area (coating surface area) with the solution is large, the method of the present invention allows high solvent removal efficiency and excellent productivity to be achieved.

### (Water-Based Liquid)

By bringing the film of the amphiphilic block polymer formed on the base member into contact with a water-based liquid, molecular assemblies of the amphiphilic polymer are obtained. The water-based liquid i water or an aqueous solution. As the aqueous solution, a biochemically and pharmaceutically acceptable aqueous solution such as distilled water for injection, physiological saline, or a buffer solution is preferably used. By including addition compounds such as a signal agent, a ligand, a drug and the like in the water-based liquid, molecular assemblies that encapsulate these addition compounds can also be obtained.

### [Molecular Assembly Production Apparatus and Outline of Processes]

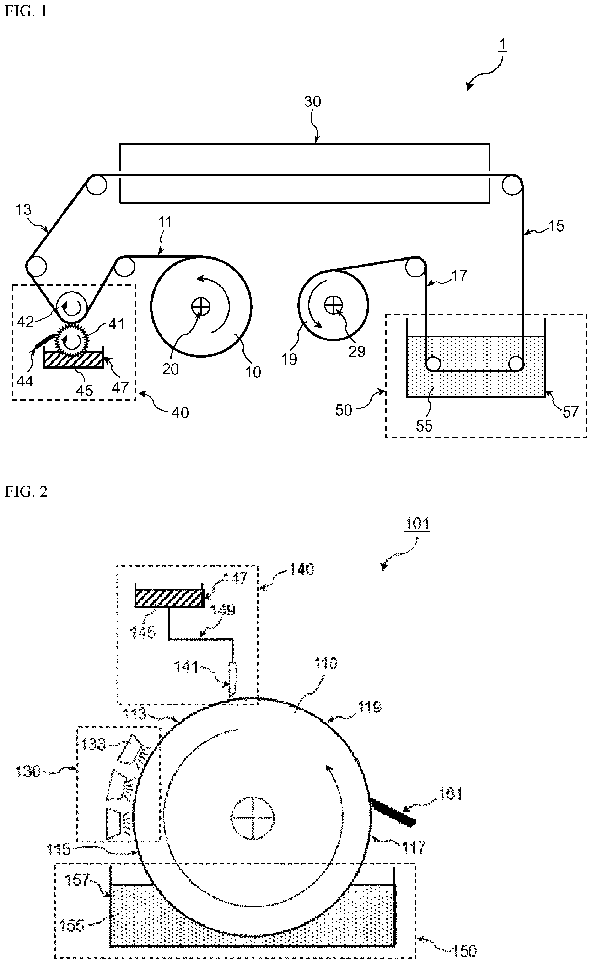

Fig. 1 is a schematic cross-sectional view illustrating a molecular assembly production apparatus used in producing molecular assemblies according to the method of the invention. In an apparatus 1 of Fig. 1, a flexible long film is used as a base member. A feeding part 20 and a winding part 29 as a base member moving mechanism are structured to be rotatable. A film base member fed out from the feeding part 20 is continuously conveyed toward the winding part 29. Coating, drying, and contact with a water-based liquid are sequentially performed. Thereafter, the base member film is collected by the winding part 29. A nip roll (not illustrated in Fig. 1) for conveying the film may be provided between the feeding part 20 and the winding part 29.

A film base member 11 fed out from a wound body 10 of the film base member set in the feeding part 20 is continuously conveyed from the feeding part 20 toward a downstream side of a base member conveying path, and is conveyed to a film forming part 40 through a guide roller. In the film forming part 40, an amphiphilic block polymer solution 45 is applied in a layered shape on the film base member. The film base member 13 on which a coated layer of the polymer solution is formed is conveyed to a drying part 30. By removing the solvent by drying, a film of the amphiphilic polymer is formed on the film base member. The film base member 15 on which the amphiphilic polymer film is formed is conveyed to a molecular assembly forming part 50, and is immersed in a water-based liquid 55, and thereby, the amphiphilic polymer film peels off from the film base member and molecular assemblies are formed in the water-based liquid. Thereafter, the film base member 17 is collected as a wound body 19 by the winding part 29.

### (Film Base Member)

A film base member is according to the invention is flexible. A film base member having excellent mechanical strength, thermal stability and solvent resistance is preferably used. As a film base member, a resin film, a metal foil, a flexible glass, or the like is used. Among these, the resin film is inexpensive, excellent in surface smoothness and is less likely to generate foreign substances, and thus, is preferable. Examples of a resin material that forms a film base member include: polyesters such as polyethylene terephthalate (PET) and polyethylene naphthalate (PEN); cellulose based polymers such as diacetyl cellulose (DAC) and triacetyl cellulose (TAC); acrylic polymers such as polymethyl methacrylate (PMMA); styrene-based polymers such as polystyrene and acrylonitrile-styrene copolymer (SAN); polyolefins such as polyethylene, polypropylene, ethylene-propylene copolymer, and trimethylpentene (PMP); cyclic polyolefins such as polynorbornene; amide-based polymers such as nylon and aromatic polyamide; polycarbonate; vinyl chloride; imide type polymer; fluorine-based polymers such as polytetrafluoroethylene (PTFE), perfluoroalkoxyalkane (PFA), and ethylene-tetrafluoroethylene copolymer (ETFE); sulfone-based polymers such as polyethersulfone; polyether ether ketone; polyphenylene sulfide; vinylidene chloride; epoxy type polymer; and the like. From these, an appropriate material is selected by taking into consideration of durability of the polymer solution with respect to the organic solvent, a coating property of the solution (such as surface wettability), heat resistance at a drying temperature, and the like.

The film base member may be colorless and transparent, or may be colored or opaque. A surface of the film base member may be subjected to an easy adhesion treatment, a release treatment, an antistatic treatment, an anti-blocking treatment, or the like. Further, for a purpose of preventing blocking or the like, an end portion in a width direction of the film base member may be subjected to embossing (knurling) or the like.

A thickness of the film base member is not particularly limited as long as the film base member is both self-supportive and flexible. The thickness of the film base member is generally about 20 µm to 300 µm, and is preferably 30 µm to 200 µm, and more preferably 35 µm to 100 µm.

### (Film Forming Part)

In the film forming part 40, the amphiphilic block polymer solution 45 is applied in a layered shape on a coating surface of the film base member 11 conveyed from the feeding part 20. Fig. 1 illustrates an embodiment (direct gravure method) in which a gravure roll 41 is brought into direct contact with the amphiphilic block polymer solution 45 in a solution pan 47. The solution 45 in the solution pan 47 adheres to a surface of the gravure roll, an excess portion of the solution on the surface of the gravure roll is scraped off by a doctor blade 44, and the amphiphilic block polymer solution is supplied to between the film base member and the gravure roll.

The film base member is conveyed while being in contact with a backup roll 42, and the solution supplied to between the gravure roll 41 and the film base member is applied in a layered shape on the base member. In this way, by bringing a side (back side) opposite to the coating surface of the flexible film base member into contact with the backup roll, the solution is applied onto the film base member of which the coating surface has a convex curved surface shape.

In the conventional method in which a solution is loaded into a container to form an amphiphilic block polymer film on an inner wall of the container, since the inner wall surface of the container is concave, solution accumulation occurs, and polymer precipitation is likely to occur during a process in which the solution is concentrated. In contrast, when the coating surface is planar or convex, the solution can be applied in a layered shape on the base member. Therefore, an amount of the solution staying in a specific region such as a bottom of a container decreases, the solvent can be dried in a short time, and polymer precipitation associated with concentration of the solution is unlikely to occur. In particular, when the coating is performed while the base member is continuously moved, a film thickness of the coated layer can be made uniform, retention of the solution in the specific region can be suppressed, and polymer precipitation can be prevented. Therefore, molecular assemblies having a uniform particle size can be easily obtained.

The gravure roll 41 and the coating surface of the film base member may be in direct contact with each other or may have a gap therebetween. The gap between the gravure roll and the film base member is preferably, for example, about 0.1 µm to 10 µm. Since the gravure roll has an uneven pattern on its surface, the gap can be adjusted to a desired range depending on a height of a convex portion on the surface of the gravure roll.

A contact surface area of the polymer solution with outside air is increased immediately after the polymer solution is applied on the base member. Therefore, an evaporation rate rapidly increases, and a solution temperature and a base member temperature tend to decrease due to that heat of vaporization is deprived. A rapid decrease in temperature may lead to problems such as polymer precipitation due to a decrease in polymer solubility in the solution and dew condensation on a film surface. In order to prevent such problems due to a rapid decrease in temperature, the film base member 11 supplied to the film forming part may be warmed. For example, film formation can be performed in a state in which the film base member is heated using a method in which the base member is heated before being conveyed to the film forming part, or using a method in which the backup roll 42 is heated. The heating of the backup roll can be performed, for example, using a method in which a heat medium such as warm water or silicone oil inside the roll is circulated, or using an electric heater or the like.

The film forming method in the film forming part 40 is not limited to the Gravure coating. Various methods such as knife roll coating, kiss roll coating, gravure coating, reverse coating, spray coating, meyer bar coating, air knife coating, curtain coating, lip coating, die coating, spin coating, dropping, and the like can be used. A coating thickness in the film forming part is not particularly limited, and can be set by taking into consideration of a thickness after drying of the amphiphilic polymer film. The thickness after drying is also not particularly limited. However, when the thickness is excessively small, peeling may occur during drying. When the thickness is excessively large, a residual solvent amount may increase due to a decrease in solvent removal efficiency during drying, and deterioration in self-accumulation due to a decrease in a contact amount with a liquid per unit area of the film in the water-based liquid may occur. Therefore, the thickness after drying is preferably about 0.5 µm to 200 µm, and more preferably about 1 µm to 100 µm.

### (Drying Part)

The film base member 13 on which a coated layer of the polymer solution is formed is conveyed to the drying part 30, the solvent is removed, and a laminate in which an amphiphilic polymer film is adhesively laminated on the film base member is obtained. In the drying part 30, it is preferable to remove the solvent by heating. Heating may be performed from either the coating surface side or a back surface side. Further, heating can also be performed from both the coating surface side and the back surface side. When the thickness of the coated layer is large, in order to reliably remove the solvent near an interface with the base member, it is preferable to heat at least from the back surface side.

A heating temperature (drying temperature) is not particularly limited as long as the temperature is lower than a heat resistant temperature of the film base member. In order to increase the solvent removal efficiency, the drying temperature is preferably equal to or higher than the boiling point of the organic solvent. The drying temperature is set, for example, in a range of about 40 to 200 °C. The drying temperature can be adjusted using appropriate heating means such as an air circulation type constant temperature oven in which hot air or cold air circulates, a heater using microwave or far infrared rays, a heated roll for temperature control, a heat pipe roll, and the like.

The temperature in a drying furnace in the drying part is not necessary to be constant throughout the entire furnace, but may have a temperature profile in which the temperature rises or decreases stepwise. For example, inside of the furnace can be divided into multiple zones and a set temperature of each zone can be varied. When the temperature in the entire drying oven is not constant, the term "drying temperature" refers to an ambient temperature in the furnace at a portion where the temperature is highest.

A drying time in the drying part is not particularly limited. From a point of view of increasing productivity, it is preferable that the drying time be as short as possible within a range in which the solvent can be sufficiently removed. As described above, in the present invention, since the solution is applied in a layered shape, the drying time can be shortened. The drying time can be adjusted by a length (furnace length) of the conveying path of the base member in the heating furnace and a conveying speed of the base member.

### (Molecular Assembly Forming Part)

The film base member 15 on which the amphiphilic polymer film is adhesively laminated is conveyed to the molecular assembly forming part 50. In the molecular assembly forming part, the water-based liquid 55 in a water bath 57 and the amphiphilic polymer film are in contact with each other, and thereby, molecular assemblies are formed. In the molecular assembly forming part 50, in order to promote the formation of the molecular assemblies, the base member on which the amphiphilic polymer film is adhesively laminated is preferably immersed in the water-based liquid 55.

When the amphiphilic polymer film and the water-based liquid are in contact with each other, in order to promote the formation of the molecular assemblies, it is preferable to perform a heating treatment or an ultrasonic treatment. Through these treatments, during a process in which the amphiphilic polymer film peels off from the base member, the molecular assemblies are formed. The heating treatment can be performed, for example, at 70 to 100 °C for 5 to 60 minutes. It is also possible that the amphiphilic block polymer film is peeled off from the base member in advance, and the peeled amphiphilic block polymer film is brought into contact with the water-based liquid.

The film base member 17 after the amphiphilic block polymer film is peeled off in the molecular assembly forming part 50 is collected as needed by being wound on the wound body 19 by the winding part 29. The collected wound body can be reused. An appropriate drying means or wiping means (not illustrated in Fig. 1) may be provided between the molecular assembly forming part 50 and the winding part 29 to remove the water-based liquid attached to the surface of the base member.

### [Other Structural Examples of Molecular Assembly Production Apparatus]

A structure of the molecular assembly production apparatus used in the production method of the present invention is not limited to the embodiment illustrated in Fig. 1 as long as the structure include: film forming part in which a solution is applied in a layered shape on a base member; a drying part in which an amphiphilic polymer film is formed on the base member by removing a solvent from the coated layer on the base member; and a molecular assembly forming part in which molecular assemblies are formed by bringing the amphiphilic polymer film into contact with a water-based liquid, and a movement mechanism is provided for sequentially moving the base member between these members.

In the embodiment illustrated in Fig. 1, the film base member is continuously conveyed from the drying part 30 to the molecular assembly forming part 50. However, it is also possible that, after drying, the film base member on which the amphiphilic polymer film is adhesively laminated is once wound on a wound body, and then, is fed out from the wound body and conveyed to the molecular assembly forming part. In this way, when the film forming and drying process and the molecular assembly forming process are discontinuous, the processes can be respectively performed at processing speeds suitable for the processes. Further, in order to more reliably remove the solvent in the amphiphilic polymer film, it is also possible that the amphiphilic polymer film is peeled off from the laminate of the amphiphilic polymer film and the film base member, and is further dried, and thereafter, the amphiphilic polymer film is supplied to the molecular assembly forming part.

Fig. 1 illustrates an example in which a flexible film base member is used. However, as disclosed herein, a rigid plate-shaped base member or a cylindrical base member can also be used. When a rigid plate-shaped base member or a flexible plate-shaped base member cut to a predetermined size is used, it is possible that the processes are sequentially performed while the base member is continuously conveyed, or the base member is moved for each process. For example, after a coated layer is formed by applying the amphiphilic block polymer solution on the base member using an applicator, the base member is moved to a heating oven or the like and is dried, and thereafter, the base member is immersed in the water-based liquid, and thereby, the molecular assemblies are obtained. Using a method in which the base member is placed on a conveyor or the like, the base member can be sequentially moved to the film forming part, the drying part, and the molecular assembly forming part.

Fig. 2 is a schematic cross-sectional view illustrating a molecular assembly production apparatus in which a cylindrical base member is used. The apparatus 101 of Fig. 2 has a cylindrical film forming drum 110 as a base member. The film forming drum 110 is rotatable, and by rotating the film forming drum, a surface of the film forming drum sequentially moves to a film forming part 140, a drying part 130, and a molecular assembly forming part 150. As a result, all of coating, drying, and molecular assembly formation, are performed on the film forming drum 110.

The film forming drum 110 is formed of resin, metal, or the like having solvent resistance with respect to an organic solvent of an amphiphilic block polymer solution 145. The film forming drum 110 is preferably temperature-adjustable by having a tube for circulating a heating medium such as warm water or silicone oil, or having an electric heater or the like. In order to facilitate control of surface temperature, the film forming drum 110 is preferably formed of a highly heat-conductive metal such as stainless steel or copper. Further, an inorganic layer such as glass (silica) or a coating such as a resin layer can be formed on a metal surface.

In the film forming part 140, the amphiphilic block polymer solution 145 discharged from a lip 141 is applied in a layered shape on the film forming drum 110. A film forming method is not limited to lip coating, and the various coating methods described above can be used. By performing coating while the film forming drum 110 is rotated (that is, the base member is continuously moved), the solution can be continuously applied in a layered shape on a convex curved outer peripheral surface, solution accumulation can be prevented, and a film thickness of the coated layer can be made uniform. By adjusting the temperature of the film forming drum 110, a rapid decrease in temperature after the application of the solution can be prevented, and problems such as polymer precipitation due to a decrease in polymer solubility in the solution and dew condensation on a film surface can be suppressed.

The coated layer of the amphiphilic block polymer solution applied in the film forming part 140 reaches the drying part 130 by the rotation of the film forming drum 110. In the drying part, as the film forming drum 110 rotates, the solvent is dried away by heat from the film forming drum. As illustrated in Fig. 2, the drying part 130 may have a heater 133 or like for heating from the coating surface side.

The amphiphilic polymer film after the solvent is removed by the drying part 130 reaches the molecular assembly forming part 150 by the rotation of the film forming drum 110. In the molecular assembly forming part, a surface 115 of the film forming drum 110 is immersed in a water-based liquid in a water bath 157. As a result, the amphiphilic polymer film is in contact with the water-based liquid 155, and molecular assemblies are formed. In the molecular assembly forming part 150, in order to promote the formation of the molecular assemblies, it is preferable to perform a heating treatment or an ultrasonic treatment. The heating treatment may be performed by heat from the film forming drum 110 or may be performed by heating the water bath 157 to raise the temperature of the water-based liquid 155.

The surface 119 of the film forming drum 110 after the amphiphilic block polymer film is peeled off in the molecular assembly forming part 150 reaches the film forming part 140 again due to the rotation of the film forming drum 110, and the amphiphilic block polymer solution is applied. During a period in which the surface of the film forming drum 110 moves from the molecular assembly forming part 150 to the film forming part 140, it is preferable to remove the water-based liquid attached to the surface 117 of the film forming drum 110 using an appropriate wiping means such as a doctor blade 161 or a drying means such as a heater.

In Fig. 2, a film forming drum is provided as a cylindrical base member. However, an endless belt can also be used as a cylindrical base member. When an endless belt is used, the film forming part, the drying part and the molecular assembly forming part may be arranged in this order along a movement path of the belt.

In Figs. 1 and 2, an upper portion of each of the water baths 57,157 is open. However, as long as the movement or rotation of the base member is not hindered, a lid or the like can be provided on the upper portion of the water bath, and thereby, scattering and evaporation of the water-based liquids 55,155 can be suppressed. Further, by providing a stirring blade, a circulation pump, or the like to stir or circulate the water-based liquid in the water bath, a localized increase in organic solvent concentration near a base member passing path can be prevented. Further, it is also possible to perform filtration, aeration or the like of the water-based liquid in the water bath to reduce organic solvent concentration in the water bath so as to promote the formation of the molecular assemblies and improve efficiency of a post-treatment.

### [Post-Treatment]

The molecular assemblies collected in the water-based liquid may be subjected to an appropriate post-treatment. An example of a post-treatment is removal of the organic solvent. Before removing the organic solvent, an appropriate purification treatment may be performed. Examples of the purification treatment include gel filtration chromatography, filtering, ultracentrifugation, and the like. In this way, a solution or dispersion of the molecular assemblies (nanoparticles) can be obtained.

The dispersion of the nanoparticles may be directly supplied for practical use, or nanoparticle powder may be formed by removing the water-based liquid or the like by filtering, freeze drying or the like. As a freeze drying treatment method, a commonly known method can be used. For example, the dispersion of the nanoparticles is frozen using liquid nitrogen or the like and sublimed under reduced pressure to obtain a freeze-dried product of the molecular assemblies. The freeze-dried product of the molecular assemblies can be supplied for practical use as a dispersion by adding an appropriate water-based liquid as needed. As a water-based liquid to be added to the freeze-dried product, a biochemically and pharmaceutically acceptable water-based liquid such as distilled water for injection, a physiological saline, a buffer solution or the like can be appropriately selected.

These post-treatments may be performed by pumping the water-based liquid out of the water bath and separating the water-based liquid from the formation of the molecular assemblies. Further, these post-treatments can also be continuously performed with the precipitation of the molecular assemblies in the water-based liquid in the molecular assembly forming part. For example, a circulation route is connected to the water bath, the water-based liquid is circulated using a circulation pump, and the post-treatments are performed in the circulation route. Thereby, the precipitation of the molecular assemblies and the post-treatments can be continuously performed.

### [Properties and Uses of Molecular Assemblies]

The molecular assemblies obtained using the method of present invention have properties similar to molecular assemblies obtained using a conventional film method. The molecular assemblies have a particle size of, for example, 10 to 500 nm. A particle size of molecular assemblies used for molecular imaging or the like in vivo is preferably 15 nm to 200 nm, and more preferably 20 nm to 100 nm. Here, the term "particle size" refers to a particle size having a highest appearance frequency in a particle distribution, that is, a central particle size in a particle distribution. The particle size of the molecular assemblies can be measured using a dynamic light scattering (DLS) method.

As described above, the particle size of the molecular assemblies can be adjusted by the chain length of the amphiphilic block polymer, presence or absence of a hydrophobic polymer and a content of the hydrophobic polymer. Further, as illustrated in Examples below, the particle size of the molecular assemblies can also be adjusted by changing the type of the organic solvent in the amphiphilic block polymer solution. Specifically, when an organic solvent in which the amphiphilic block polymer is highly soluble is used, there is a tendency that molecular assemblies having a small and uniform particle size and a unimodal particle size distribution are obtained.

The particle size distribution of the molecular assemblies is preferably unimodal. Whether or not the particle size distribution is unimodal can be determined by visual inspection of a histogram. Further, as an indicator of the unimodality, polydispersity index (PdI) of particle sizes may be used. The PdI of particle sizes of the molecular assemblies is preferably 0.3 or less, and more preferably 0.2 or less.

As described above, in the present invention, by using a method in which a signal agent, a ligand, a drug and the like are contained in the amphiphilic block polymer solution and/or the water-based liquid, or a method in which a signal agent, a ligand, a drug and the like are bound to the amphiphilic block polymer or the hydrophobic polymer, molecular assemblies containing these substances are obtained. The molecular assemblies are used for a drug delivery system, molecular imaging, and the like. Drug delivery and molecular imaging can be performed by administering the molecular assemblies in vivo. Methods of administering the molecular assemblies in vivo include blood administration, oral administration, transdermal administration, transmucosal administration and the like. An administration subject of the molecular assemblies can be a human or a non-human animal. Examples of non-human animals include mammals other than humans, more specifically, primates, rodents (such as mice and rats), rabbits, dogs, cats, pigs, cows, sheep, horses, and the like.

The molecular assemblies having the above-described particle size are excellent in specific accumulation to vascular lesion sites (such as a malignant tumor site, an inflammation site, an arteriosclerosis site, and an angiogenesis site). Since the molecular assemblies accumulate in tissues of these sites due to an EPR (enhanced permeability and retention) effect, the accumulation of the molecular assemblies is independent of a tissue type of a vascular lesion site. Examples of administration targets include cancer diseases such as liver cancer, pancreatic cancer, lung cancer, cervical cancer, breast cancer, colon cancer and the like. Further, the molecular assemblies can also be used as substance delivery carriers in cosmetics, foods, and the like.

### Examples

In the following, the present invention is described in more detail by comparing production examples of molecular assemblies based on the method of the present invention with production examples based on a conventional method. However, the present invention is not limited to these examples.

In the following Experimental Examples and Reference Examples, the particle size and the polydispersity index (PdI) of the molecular assemblies were measured using a Zetasizer Nano S (manufactured by Malvern Co., Ltd.) using a dynamic light scattering (DLS) method. When contamination due to factors of an experimental environment was observed in the DLS measurement results (Experimental Examples 2 to 9 and 14), measurement was re-performed after passing the molecular assemblies through a 100 nm filter.

### [Synthesis Example of Amphiphilic Block Polymer]

With reference to a method described in WO 2009/148121, using sarcosine anhydride and aminated poly L-lactic acid as monomer components, a linear amphiphilic block polymer (PSar64-PLLA 31; molecular weight: 6943) that has a hydrophilic block having 64 sarcosine units and a hydrophobic block having 31 L-lactic acid units was synthesized using glycolic acid, O-(benzotriazol-1-yl)-N,N,N',N'-tetramethyluronium hexafluorophosphate (HATU) and N,N-diisopropylethylamine (DIEA).

### [Reference Example A]

The block polymer obtained in above-described Synthesis Example was dissolved in chloroform to obtain a solution of 100 mg/mL. While a glass plate was heated to 70 °C on a planar heater, 150 µL of the above-described block polymer solution was dropped in a range of about 5 cm² on the glass plate (thickness of coated layer: about 300 µm). Thereafter, heating using the planar heater was continued, and the solvent was sufficiently removed, and thereby, a block polymer film on the glass plate was obtained. The obtained polymer film was immersed in distilled water (1 mL per 1 mg of the polymer) and heating was performed at 85 °C for 20 minutes, and a dispersion of amphiphilic block polymer particles was obtained.

### [Experimental Examples 2 to 5]

In Experimental Examples 2 to 5, while a resin film to 70 °C was heated on a planar heater, 150 µL of the above-described block polymer solution was dropped onto the resin film. Similar to Reference Example A, a block polymer film was produced and was immersed in distilled water, and heating was performed, and a dispersion of amphiphilic block polymer particles was obtained. The resin films used in Experimental Examples 2 to 5 were respectively films of perfluoroalkoxyalkane (PFA; Experimental Example 2), polyethylene terephthalate (PET; Experimental Example 3), polymethylpentene (PMP; Experimental Example 4), and polyimide (PI; Experimental Example 5).

### [Reference Examples B and C and Experimental Examples 7 to 9 and 11 to 12]

The type of the organic solvent, the type of the base member and the drying temperature (heating temperature of the planar heater) were changed as shown in Table 1. Other than that, in the same manner as in Reference Example A, a dispersion of amphiphilic block polymer particles was obtained.

### [Reference Examples 1 to 3]

In Reference Examples 1 to 3, amphiphilic block polymer particles were obtained using the film method described in WO 2009/148121. Specifically, a block polymer solution was placed in a glass flask and a polymer film was formed on a wall surface of the flask using an evaporator. Further, a film was formed on the inner wall surface, water was added to the flask, and an ultrasonic treatment was performed at a temperature of 85 °C for 30 minutes, and a dispersion of amphiphilic block polymer particles was obtained. In Reference Examples 1 to 3, a synthetic lot polymer different from the above-described Experimental Examples was used. The number of sarcosine units (PSar), the number of L-lactic acid units (PLLA) and a molecular weight of a block polymer used in each of Reference Examples 1 to 3 are shown in Table 1.

Solution composition (composition of the block polymer, molecular weight, and type of the solvent), film formation conditions (type of the base member and drying temperature), and DLS measurement results (particle size and PdI) of each of the above-described Experimental Examples and Reference Examples are shown in Table 1.

**[Table 1]**

| | Solution Composition | | Film Forming Conditions | | DLS Measurement Results | |
|---|---|---|---|---|---|---|
| | Solvent | Block Polymer (Composition/Molecular Weight) | Base Member | Drying Temperature (°C) | Particle Size (nm) | PdI |
| Reference Example A | Chloroform | PSar64-PLLA31 / 6943 | Glass | 70 | 25.1 | 0.13 |
| Experimental Example 2 | Chloroform | PSar64-PLLA31 / 6943 | PFA | 70 | 26.8 | 0.09 |
| Experimental Example 3 | Chloroform | PSar64-PLLA31 / 6943 | PET | 70 | 26.9 | 0.10 |
| Experimental Example 4 | Chloroform | PSar64-PLLA31 / 6943 | TPX | 70 | 26.8 | 0.08 |
| Experimental Example 5 | Chloroform | PSar64-PLLA31 / 6943 | PI | 70 | 26.2 | 0.09 |
| Reference Example 1 | Chloroform | PSar78-PLLA30 / 7866 | Glass Container | | 30.4 | 0.16 |
| Reference Example B | DMF | PSar64-PLLA31 / 6943 | Glass | 140 | 28.9 | 0.16 |
| Experimental Example 7 | DMF | PSar64-PLLA31 / 6943 | PFA | 160 | 27.5 | 0.16 |
| Experimental Example 8 | DMF | PSar64-PLLA31 / 6943 | PET | 160 | 26.7 | 0.18 |
| Experimental Example 9 | DMF | PSar64-PLLA31 / 6943 | PI | 160 | 25.7 | 0.11 |
| Reference Example C | Methanol | PSar64-PLLA31 / 6943 | Glass | 70 | 173 | 0.42 |
| Reference Example 2 | Methanol | PSar78-PLLA30 / 7866 | Glass Container | | 168 | 0.26 |
| Experimental Example 11 | Ethanol | PSar64-PLLA31 / 6943 | PFA | 85 | 182 | 0.30 |
| Experimental Example 12 | Ethanol | PSar64-PLLA31 / 6943 | PET | 85 | 166 | 0.24 |
| Reference Example 3 | Ethanol | PSar63-PLLA30 / 6640 | Glass Container | | (188) | — |

In each of Experimental Examples 2 to 5 in which chloroform was used as the organic solvent, molecular assemblies having substantially the same particle size and having a unimodal particle size distribution with a PdI of 0.10 or less were obtained. Further, in each of Experimental Examples 7 to 9 in which DMF was used as the organic solvent, similar results were also obtained. From these results, it is clear that molecular assemblies of an amphiphilic block polymer can be obtained using the method of the present invention regardless of the type of the base member.

Further, from the above-described results, it is clear that, in the method of the present invention, regardless of the properties (hydrophobic or hydrophilic) of the base member surface, a base member having solvent resistance with respect to the organic solvent and heat resistance during heat drying can be arbitrarily used. A reason that the particle size of the molecular assemblies is not affected by the base member surface (the interface between the base member and the amphiphilic polymer layer) is because the amphiphilic polymer coated and dried on the base member does not form an integrated film such as a monomolecular film, and the molecular assemblies are formed due to a thermodynamic action when the amphiphilic polymer film as a non-aggregated body is in contact with the water-based liquid, and thus the influence of the properties of the base member surface on the collective form of the final molecular assemblies is small. Or, even when an integrated film is formed at the interface with the base member, in a layer above the integrated film, the polymer is randomly arranged and does not form an integrated film, and thus is only limitedly affected by the properties of the base member surface. By taking these results into consideration, it is clear that, even when the thickness of the amphiphilic block polymer film formed on the base member is large, molecular assemblies having small and highly uniform particle size are formed.

The particle size and PdI of the molecular assemblies obtained in each of Experimental Examples 2 to 5 were similar to those obtained in Reference Example 1 based on the conventional film method in which the molecular assemblies were formed in a glass container (in Reference Example 1, the particle size is large, but this is believed to be due to a different chain length of the amphiphilic block polymer). From this result, it is believed that the molecular assemblies obtained using the method of the present invention are equivalent to the molecular assemblies obtained using the conventional film method.

Also in Reference Example C in which methanol is used as the organic solvent and in Experimental Examples 11 and 12 in each of which ethanol is used as the organic solvent, molecular assemblies having a particle size equivalent to that based on the conventional method (Reference Examples 2 and 3) were obtained (in Reference Example 3, a maximum value of the particle size distribution is indicated in parentheses because the particle size distribution does not have unimodality, and the PdI cannot be accurately evaluated). From these results, it is clear that even when alcohols are used as the organic solvent, molecular assemblies equivalent to those based on the conventional film method are obtained.

### [Reference Examples D and E and Experimental Example 14]

In Reference Examples D and E and Experimental Example 14, a solution was used which was obtained by dissolving in chloroform a polylactic acid (linear homopolymer of DL-lactic acid having a weight average molecular weight of 5000: PLA-0005 manufactured by Wako Pure Chemical Industries, Ltd.) as a hydrophobic polymer in addition to an amphiphilic block polymer (PSar64-PLLA31). The content of the polylactic acid was 20 mol% (Reference Example D and Experimental Example 14) and 100 mol% (Reference Example E) with respect to 100 mol% of the amphiphilic block polymer. In Reference Examples D and E, while a glass plate was heated to 70 °C on a planar heater, the solution was dropped onto the glass plate. In Experimental Example 14, while a PET film was heated to 70 °C on a planar heater, the solution was dropped onto the PET film. Other than that, in the same manner as in Reference Example A, a dispersion of amphiphilic block polymer particles was obtained.

### [Reference Example 4]

In Reference Example 4, a solution containing 25 mol% of a polylactic acid (PLA0005) with respect to 100 mol% of an amphiphilic block polymer (PSar 63-PLLA 30) was used and amphiphilic block polymer particles were obtained based on the film method described in WO 2009/148121.

Solution composition, film formation conditions, and DLS measurement results of Experimental Example 14 and Reference Examples D, E and 4, together with the results of Experimental Example 3 and Reference Examples A and 1, are shown in Table 2.

**[Table 2]**

| | Solution Composition | | | Film Forming Conditions | | DLS Measurement Results | |
|---|---|---|---|---|---|---|---|
| | Solvent | Block Polymer (Composition/Molecular Weight) | PLA0005 (mol%) | Base Member | Drying Temperature (°C) | Particle Size (nm) | PdI |
| Reference Example A | Chloroform | PSar64-PLLA31 / 6943 | 0 | Glass | 70 | 25.1 | 0.13 |
| Experimental Example 3 | Chloroform | PSar64-PLLA31 / 6943 | 0 | PET | 70 | 26.9 | 0.10 |
| Reference Example 1 | Chloroform | PSar78-PLLA30 / 7866 | 0 | Glass Container | | 30.4 | 0.16 |
| Reference Example D | Chloroform | PSar64-PLLA31 / 6943 | 20 | Glass | 70 | 32.9 | 0.26 |
| Experimental Example 14 | Chloroform | PSar64-PLLA31 / 6943 | 20 | PET | 70 | 38.0 | 0.21 |
| Reference Example 4 | Chloroform | PSar63-PLLA30 / 6640 | 25 | Glass Container | | 38.1 | 0.17 |
| Reference Example E | Chloroform | PSar64-PLLA31 / 6943 | 100 | Glass | 70 | 78.4 | 0.17 |

In Reference Example D and Experimental Example 14, by adding a polylactic acid as a hydrophobic polymer, the particle size of the molecular assemblies was increased as compared to Reference Example A and Experimental Example 3. A similar trend was observed in a comparison between Reference Example 1 and Reference Example 4. Further, in Reference Example E having a large blending amount of the polylactic acid, the particle size was even larger. From these results, it is clear that, even in the method of the present invention, by incorporating, in addition to the amphiphilic block polymer, the hydrophobic polymer in the solution, the particle size of the molecular assemblies can be continuously controlled.

### [Description of Reference Numerals]

1, 101: molecular assembly production apparatus
10, 11, 13, 15, 17, 19: film base member
110: film forming drum
40, 140: film forming part
41: gravure roll (film forming roll)
42: backup roll
45, 145: amphiphilic block polymer solution
30, 130: drying part
50, 150: molecular assembly forming part
55, 155: water-based liquid
57, 157: water bath

## Claims

1. A molecular assembly production method for producing molecular assemblies of an amphiphilic block polymer, comprising:
applying a polymer solution in a layered shape on a planar base member, wherein the base member is a flexible film, the polymer solution containing an amphiphilic block polymer and a solvent, the amphiphilic block polymer having a hydrophilic block chain having 20 or more sarcosine units and a hydrophobic block chain having 10 or more lactic acid units;
forming a polymer film on the base member by removing the solvent from the coated layer of the polymer solution; and
obtaining molecular assemblies by immersing the base member in a water-based liquid.

2. The molecular assembly production method according to claim 1, wherein, when the polymer solution is applied in a layered shape, the base member has a planar shape or a coating surface of the base member has a convex curved surface shape.

3. The molecular assembly production method according to claim 1 or 2, wherein the polymer solution further contains a hydrophobic polymer, optionally wherein the hydrophobic polymer is a polymer to which at least one selected from a group consisting of a signal agent, a ligand and a drug is bound.

4. The molecular assembly production method according to any one of claims 1 to 3, wherein
the polymer solution contains at least one addition compound selected from a group consisting of a signal agent, a ligand and a drug, and
the molecular assemblies are molecular assemblies containing the amphiphilic block polymer and the addition compound contained in the polymer solution.

5. The molecular assembly production method according to any one of claims 1 to 4, wherein the solvent of the polymer solution has a boiling point of 200 °C or less.

6. The molecular assembly production method according to any one of claims 1 to 5, wherein
the water-based liquid contains at least one addition compound selected from a group consisting of a signal agent, a ligand and a drug, and
the molecular assemblies are molecular assemblies containing the amphiphilic block polymer and the addition compound contained in the water-based liquid.

7. The molecular assembly production method according to any one of claims 1 to 6, wherein the polymer solution is applied in a layered shape on the base member from a gravure roll.

## Patentansprüche

1. Molekülassemblatherstellungsverfahren zur Herstellung von Molekülassemblaten eines amphiphilen Blockpolymers, das Folgendes umfasst:
Auftragen einer Polymerlösung in Schichtform auf ein planares Basiselement, wobei das Basiselement ein flexibler Film ist, wobei die Polymerlösung ein amphiphiles Blockpolymer und ein Lösungsmittel enthält, wobei das amphiphile Blockpolymer eine hydrophile Blockkette mit 20 oder mehr Sarcosineinheiten und eine hydrophobe Blockkette mit 10 oder mehr Milchsäureeinheiten aufweist,
Ausbilden eines Polymerfilms auf dem Basiselement durch Entfernen des Lösungsmittels aus der Beschichtungsschicht der Polymerlösung; und
Erhalten von Molekülassemblaten durch Eintauchen des Basiselements in eine Flüssigkeit auf Wasserbasis.

2. Molekülassemblatherstellungsverfahren nach Anspruch 1, wobei, wenn die Polymerlösung in Schichtform aufgetragen wird, das Basiselement eine planare Form oder eine Beschichtungsoberfläche des Basiselements eine konvexe gekrümmte Oberflächenform aufweist.

3. Molekülassemblatherstellungsverfahren nach Anspruch 1 oder 2, wobei die Polymerlösung weiters ein hydrophobes Polymer enthält, wobei das hydrophobe Polymer gegebenenfalls ein Polymer ist, an das zumindest eines gebunden ist, das aus der aus einem Signalmittel, einem Liganden und einem Arzneimittel bestehenden Gruppe ausgewählt ist.

4. Molekülassemblatherstellungsverfahren nach einem der Ansprüche 1 bis 3, wobei
die Polymerlösung zumindest eine zusätzliche Verbindung enthält, die aus der aus einem Signalmittel, einem Liganden und einem Arzneimittel bestehenden Gruppe ausgewählt ist, und
die Molekülassemblate Molekülassemblate sind, die das amphiphile Blockpolymer und die Additionsverbindung enthalten, die in der Polymerlösung enthalten ist.

5. Molekülassemblatherstellungsverfahren nach einem der Ansprüche 1 bis 4, wobei das Lösungsmittel der Polymerlösung einen Siedepunkt von 200 °C oder weniger aufweist.

6. Molekülassemblatherstellungsverfahren nach einem der Ansprüche 1 bis 5, wobei
die Flüssigkeit auf Wasserbasis zumindest eine Additionsverbindung enthält, die aus der aus einem Signalmittel, einem Liganden und einem Arzneimittel bestehenden Gruppe ausgewählt ist, und
die Molekülassemblate Molekülassemblate sind, die das amphiphile Blockpolymer und die Additionsverbindung enthalten, die in der Flüssigkeit auf Wasserbasis enthalten ist.

7. Molekülassemblatherstellungsverfahren nach einem der Ansprüche 1 bis 6, wobei die Polymerlösung von einer Gravurwalze in Schichtform auf das Basiselement aufgetragen wird.

## Revendications

1. Procédé de production d'ensembles moléculaires pour produire des ensembles moléculaires d'un polymère séquencé amphiphile, comprenant les étapes consistant à :
appliquer une solution polymère sous une forme stratifiée sur un élément de base plan, dans lequel l'élément de base est un film flexible, la solution polymère contenant un polymère séquencé amphiphile et un solvant, le polymère séquencé amphiphile ayant une chaîne séquencée hydrophile ayant 20 unités de sarcosine ou plus et une chaîne séquencée hydrophobe ayant 10 unités d'acide lactique ou plus ;
former un film polymère sur l'élément de base en éliminant le solvant de la couche revêtue de la solution polymère ; et
obtenir des ensembles moléculaires en immergeant l'élément de base dans un liquide à base d'eau.

2. Procédé de production d'ensembles moléculaires selon la revendication 1, dans lequel lorsque la solution polymère est appliquée sous une forme stratifiée, l'élément de base a une forme plane ou une surface de revêtement de l'élément de base a une forme de surface incurvée convexe.

3. Procédé de production d'ensembles moléculaires selon la revendication 1 ou 2, dans lequel la solution polymère contient en outre un polymère hydrophobe, facultativement dans lequel le polymère hydrophobe est un polymère dont au moins un choisi dans un groupe constitué d'un agent de signalisation, d'un ligand et d'un médicament est lié.

4. Procédé de production d'ensembles moléculaires selon l'une quelconque des revendications 1 à 3, dans lequel
la solution polymère contient au moins un composé d'addition choisi dans un groupe constitué d'un agent de signalisation, d'un ligand et d'un médicament, et
les ensembles moléculaires sont des ensembles moléculaires contenant le polymère séquencé amphiphile et le composé d'addition contenu dans la solution polymère.

5. Procédé de production d'ensembles moléculaires selon l'une quelconque des revendications 1 à 4, dans lequel le solvant de la solution polymère a un point d'ébullition de 200°C ou moins.

6. Procédé de production d'ensembles moléculaires selon l'une quelconque des revendications 1 à 5, dans lequel
le liquide à base d'eau contient au moins un composé d'addition choisi dans un groupe constitué d'un agent de signalisation, d'un ligand et d'un médicament, et
les ensembles moléculaires sont des ensembles moléculaires contenant le polymère séquencé amphiphile et le composé d'addition contenu dans le liquide à base d'eau.

7. Procédé de production d'ensembles moléculaires selon l'une quelconque des revendications 1 à 6, dans lequel la solution polymère est appliquée sous une forme stratifiée sur l'élément de base à partir d'un rouleau de gravure.
